# EUROPÄISCHE PATENTSCHRIFT

(11) **EP 0 540 525 B1**
(45) Veröffentlichungstag und Bekanntmachung des Hinweises auf die Patenterteilung: **22.09.1999**
(21) Anmeldenummer: 91909986.1
(22) Anmeldetag: 24.05.1991
(51) Int. Cl.: C12N 15/33, C12N 1/06, C12P 21/08, A61K 39/02

(54) **VERFAHREN ZUR HERSTELLUNG VON VAKZINEN UND IHRE VERWENDUNG**
METHOD OF PREPARATION OF VACCINES, AND THE USE OF THE VACCINES THUS PREPARED
PROCEDE POUR LA FABRICATION DE VACCINS ET LEUR UTILISATION

(30) Priorität: 26.07.1990 DE 4023721
(43) Veröffentlichungstag der Anmeldung: 12.05.1993
(73) Patentinhaber: EVAX Technologies AG, 82152 Martinsried/München (DE)
(72) Erfinder: Lubitz, Werner, Prof. Dr., 1090 Wien (AT)
(74) Vertreter: Huber, Bernhard, Dipl.-Chem.
(86) Internationale Anmeldenummer: EP9100967
(87) Internationale Veröffentlichungsnummer: WO9201791

(56) Entgegenhaltungen:
- EP-A- 140 864
- EP-A- 291 021
- WO-A-91/13155
- GB-A- 2 143 238
- RESEARCH IN MICROBIOLOGY, Volume 141, 6 June 1990, INSTITUT PASTEUR/ELSEVIER, SZOSTAK M. et al., "Recombinant Bacterial Ghosts as Vaccines", pages 1005-1008.
- BIOCHIMIE, Volume 72, No. 2-3, March 1990, ELSEVIER, Paris, WITTE A. et al., "PhiX174 Protein E-mediated Lysis of Escherichia Coli", pages 191-200.
- EUROPEAN JOURNAL OF BIOCHEMISTRY, Volume 180, 1989, WITTE A. & LUBITZ W., "Biochemical Characterization of PhiX174-protein-E-mediated Lysis of Escherichia Coli", pages 393-398.

## Beschreibung

Die Erfindung betrifft ein Verfahren zur Herstellung von Vakzinen.

Die Hauptaufgabe des Immunsystems bei Mensch und Tier besteht in der Abwehr und Vermeidung pathologischer Schäden, die aufgrund von entarteten Zellen, infektiösen Viren, Bakterien, Pilzen oder Protozoen entstehen. Das Immunsystem zeichnet sich dadurch aus, daß eine immer stärker werdende Resistenz nach wiederholten Infekten mit Krankheitserregern auftritt. Ziel der Immunisierung ist es, Abwehrkräfte des Immunsystems gegen bestimmte Krankheitserreger aufzubauen, ohne entsprechende Krankheiten auszulösen.

Antikörper und zelluläre T- und B-Lymphozyten, sorgen für die spezifische Abwehr von Erregern. Dabei ist die Erkennung fremder Strukturen wie z.B. solcher, die auf einer Bakterienzelle vorkommen, eine wesentliche Voraussetzung. Je nach Stimulierung des Immunsystems kann dabei nach Immunisierung eine zeitlich begrenzte oder eine lebenslange Immunität gegen Krankheitserreger aufgebaut werden.

Für die Wirksamkeit von Vakzinen ist es wesentlich, daß die Immunantwort in ausreichendem Umfang erfolgt. Aus diesem Grund verwendet man vorteilhaft als Immunogene Substanzen, die in ihrer Zusammensetzung und in ihrem Aufbau dem Erreger, gegen den Immunität erreicht werden soll, weitgehend gleichen. So werden abgeschwächte bzw. abgetötete Bakterien oder Viren, aufbereitete Teilkomponenten von Krankheitserregern (Membranproteine von Bakterien, Strukturproteine von Viren) oder rekombinante Lebenvakzine (Viren oder Bakterien) eingesetzt. Ein Nachteil der Verwendung von lebenden Bakterien oder Viren als Immunogene liegt darin, daß eine unerwünschte krankheitserregende Ausbreitung der Keime nicht völlig ausgeschlossen werden kann. Durch Abtötung oder Fragmentierung der Bakterien und Viren vor Verwendung als Immunogen oder Vakzine kann diese Gefahr vermindert werden. Allerdings besteht das Risiko, daß die antigenen Determinanten verändert werden, wodurch die Immunantwort deutlich geringer sein kann.

In der eigenen älteren Anmeldung WO-A-91/13155 wird die Verwendung eines modifizierten gramnegativen Bakterium als Adjuvanz vorgeschlagen, welches durch Transformation mit einem Gen, welches für ein Fusionsprotein codiert das aus einem lytischen Membranprotein und einer hydrophoben, nicht lytischen membranintegrierenden Proteindomäne besteht und Expression des Gens unter Freisetzung der cytoplasmatischen Bestandteile unter Bildung eines Bakterienghosts erhalten wird. Dieser Bakterienghost enthält jedoch das rekombinante Protein in seiner Zellwand integriert, so daß nicht mehr die ursprüngliche Bakterienzellwand vorliegt sondern letztere den Träger des rekombinanten Proteins darstellt.

Aufgabe der vorliegenden Erfindung ist es deshalb, gegen gramnegative Bakterien, die Krankheitserreger sein können, Immunogene und Vakzine bereitzustellen, die diese Nachteile nicht besitzen.

Diese Aufgabe wird durch ein Verfahren zur Herstellung eines Vakzins, gelöst, bei dem man ein gramnegatives Bakterium, welches kein Fusionsproteingen enthält, das für eine hydrophobe, nicht lytisch wirkende membranintegrierende Proteindomäne und ein rekombinates Protein codiert mit einem Gen eines lytisch wirkenden Membranproteins aus Bakteriophagen, mit einem lytisch wirkenden Toxin-Releasgen oder mit Genen, die Teilsequenzen davon enthalten, die für lytische Proteine codieren, transformiert, das Bakterium kultiviert, das Lyse-Gen exprimiert und anschließend das so modifizierte Bakterium, dessen cytoplasmatische Bestandteile freigesetzt worden sind, wobei die Integrität des Zellwandkomplexes weitgehend erhalten bleibt, aus der Kulturbrühe isoliert.

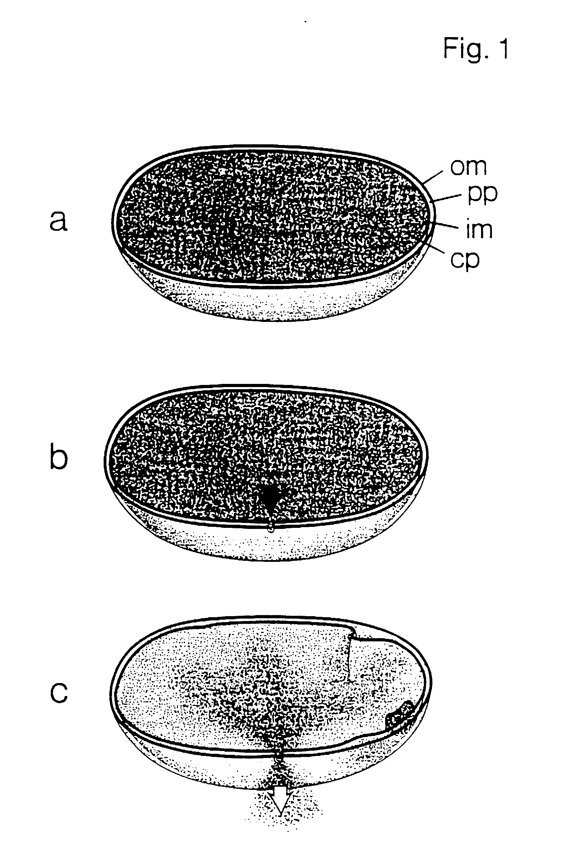

Die Expression des Lyse-Gens erfolgt vorzugsweise verzögert bei der Fermentation während des Zellwachstums. Dadurch wird erreicht, daß zunächst eine ausreichende Menge von Bakterien entsteht, bevor die Lyse dieser Bakterien erfolgt. Der sonst dichte Zellwandkomplex der Bakterien wird dabei so permeabilisiert, daß die cytoplasmatischen Bestandteile der Bakterien freigesetzt werden. (Eur. J. Biochem. 180 (1989), 393 - 398). Die Morphologie der Zellen, beispielsweise die Stäbchenform von E.coli-Zellen, bleibt erhalten. Es bildet sich lediglich in einem abgegrenzten Bereich der Membran eine Tunnelstruktur aus. Die Tunnelbildung wird begleitet durch eine Fusion der inneren und äußeren Membran am Tunnelrand. Die auf diese Weise entstandenen modifizierten Bakterien werden im weiteren als Bakterienghosts bezeichnet. Bakterienghosts und ihre Herstellung sind beispielsweise beschrieben in Eur. J. Biochem. 180 (1989) 393 - 398, Biochimie 72 (1990) 191 -200 und J. Bacteriol. 172 (1990) 4109 - 4114. Ihren schematischen Aufbau zeigt Fig. 1.

Die Bakterienghosts bestehen aus cytoplasmatischer (innerer) Membran, periplasmatischem Raum und äußerer Membran, wobei die Integrität des Zellwandkomplexes weitgehend erhalten bleibt. Für Bakterienstämme, die zusätzlich eine S-Layer-Schicht (parakristalline Proteinschicht außerhalb der äußeren Membran) aufweisen, ist diese Proteinschicht ebenfalls Bestandteil der Bakterienghosts (Ann. Rev. Microbiol. 37 (1983), 311 - 339).

Als Bakterien sind alle gramnegativen Bakterien, vorzugsweise gramnegative Krankheitserreger z.B. der Genera Neisseria, Escherichia, Bordetella, Campylobacter,Legionella, Pseudomonas, Shigella, Vibrio, Yersinia, Salmonella, Haemophilus, Brucella, Francisella und Bacterioides geeignet (Schaechter, M, H. Medoff, D. Schlesinger, Mechanisms of Microbial Disease. Williams and Wilkins, Baltimore (1989)).
Beispiele für pathogene E.coli-Stämme sind:
ATCC Nr. 31618, 23505, 43886, 43892, 35401, 43896, 33985, 31619 und 31617.

Die Bakterienghosts eignen sich überraschend gut als Immunogene, wobei es zu ausgeprägten zellulären und humoralen Immunantworten kommt.

Ein weiterer Vorteil der erfindungsgemäßen Bakterienghosts besteht darin, daß sehr viele antigene Epitope des Zellwandkomplexes durch die Bakterienghosts präsentiert werden. Außerdem wirkt das in den Bakterienhüllen vorhandene Lipopolysaccharid als Mitogen und löst ebenfalls ein Signal zur Zellteilung aus. Damit erhält man eine effektive Stimulation der B-Zell-spezifischen Produktion von Immunglobulinen.

Unter lytisch wirkenden Membranproteinen aus Bacteriophagen sind vorzugsweise Membranproteine von Bacteriophagen der Klasse Mikroviridae, vorzugsweise von icosahedralen, lytischen und ssDNA enthaltenden Phagen, die Enterobacteriacae infizieren können, zu verstehen. Beispiele hierfür sind die Phagen PhiX174, S13, G4, G6,

G14, PhiA, PhiB, PhiC, PhiR, welche E. coli C Stämme infizieren können. Ebenso geeignet ist alpha3, welcher E. coli C und E. coli B Stämme infizieren kann. Ebenso geeignet sind die Phagen K9, St-1, PhiK, PhiXtB und U3, welche E. coli K12 Stämme infizieren können ( Sinsheimer R.L. (1968) in: Prog. Nucl. Acid Res. Mol. Biol (Davidson J.N. & Cohn W.W., eds) Vol.8, Academic Press, New York & London, pp. 115-169; Tessman E.S. & Tessmann I. (1978) in: The single-stranded DNA Phages (Denhardt D.T., Dressler D. & Ray D.S., eds.) Cold Spring Harbor Press, Cold Spring Harbor, pp. 9-29; Hayashi M., Aoyama A., Richardson D.L. & Hayashi M.N. (1987) in: the Bacteriophages, (Calendar R., ed.) Plenum Press, New York, pp. 1-71).

Die Herstellung von Genen, die Teilsequenzen von lytischen Proteinen oder Toxin-Releasegenen enthalten, erfolgt vorzugsweise nach Methoden des Genetic Engineering über Protein Engineering, Protein Design oder Protein Redesign wie sie beispielsweise in D.L. Oxender, C.F. Fox 'Protein Engineering' A.R. Liss, Inc. New York, 1987 beschrieben sind.

In einer bevorzugten Ausführungsform enthält das Lyse-Gen die DNA-Sequenz des E-Proteins, die N-terminale, membrandurchdringende Domäne des E-Proteins, die DNA-Sequenz des L-Proteins, die C-terminale, membrandurchdringende Domäne des L-Proteins oder die DNA-Sequenz des EL-Hybridproteins (Sequenzen vgl. EP-A 0 291 021. Ebenso geeingnet sind Teilsequenzen davon, die lytisch wirken. Als lytisch wirksame Membranproteine sind ebenfalls bevorzugt Lyseproteine aus den genannten Bakteriophagen sowie andere Toxin-release Gene wie das Colicin Lysegen (Microbiol. Sciences 1 (1984) 168-175 und 203 -205).

Die Transformation durch einen Vektor und die Expression der Plasmid-codierten Gene kann nach den dem Fachmann gelaufigen Verfahren erfolgen. Vorzugsweise erfolgt die Transformation durch Elektroporation oder Konjugation. Weitere Angaben zu geeigneten Lyse-Genen und Vektoren, zur Transformation, Expression und Lyse finden sich in Witte A. und Lubitz W., Eur. J. Biochem. 180 (1989) 393 - 398 sowie den dort angegebenen Referenzen. Im übrigen entsprechen die bevorzugten Ausführungsformen dieses Verfahrens den bevorzugten Ausführungsformen der erfindungsgemäßen Vakzine.

Vorzugsweise wird während der Wachstumsphase bei der Fermentation zunächst die Aktivität des Lytischen Proteins inhibiert oder die Expression des Lysegens reprimiert und erst zu einem gewünschten Zeitpunkt, vorzugsweise in der späten logarithmischen Phase, die Inhibierung oder Repression aufgehoben (vorzugsweise wird zur Inhibierung ein Erdalkalisalz, wie z. B. Magnesiumsulfat, zugesetzt. Der bevorzugte Konzentrationsbereich beträgt 0,1 - 0,6 mol/l).

Das erfindungsgemäß hergestellte Vakzin eignet sich zur Herstellung von Antikörpern, indem ein Säugetier mit einem erfindungsgemäß modifizierten Bakterium immunisiert wird und die Antikörper nach bekannten Verfahren, z.B. aus dem Serum oder der Milz gewonnen werden.

Hierzu werden zweckmäßig B-Lymphozyten der immunisierten Tiere in Gegenwart von transformierenden Agenzien mit einer geeigneten Zellinie fusioniert, die Zellinie, welche die gewünschten Antikörper produziert, kloniert und kultiviert und aus den Zellen oder dem Kulturüberstand die monoklonalen Antikörper gewonnen.

Die erfindungsgemäßen Vakzine können auch zur Stimulierung von T-Lymphozyten und als Adjuvans verwendet werden sowie zur Herstellung von Impfstoffen. Die Herstellung dieser Impfstoffe kann nach den bekannten Methoden durchgeführt werden. Bevorzugt werden jedoch die Ghosts zunächst lyophilisiert und anschließend, ggf. unter Zusatz von Hilfsstoffen, suspendiert.

Es ist weiter bevorzugt, das Vakzin als multivalentes Vakzin zu formulieren. Hierzu kann das erfindungsgemäße Vakzim mit Vakzinen, wie sie in der DE 40 05 874.3 beschrieben sind, kombiniert werden. Ebenso ist eine Kombination mit sonstigen, dem Fachmann geläufigen Vakzinen möglich. Dabei kann das erfindungsgemäße Vakzin als Vakzin oder als Adjuvans wirken.

In einer bevorzugten Ausführungsform wird das Vakzin als Suspension von Bakterienghosts in einer antigenhaltigen Lösung appliziert. Dabei ist es bevorzugt, durch geeignete Maßnahmen, beispielsweise durch Suspendieren der gefriergetrockneten Bakterienghosts in dieser antigenhaltigen Lösung, die Antigene ins Innere der Bakterienghosts einzubringen.

In einer weiteren bevorzugten Ausführungsform enthält das Vakzin noch einen Anteil von 0,01 % bis 5 %, vorzugsweise 0,01 % bis 2 % und besonders bevorzugt 0,01 % - 1 % an lebenden Bakterien (bezogen auf die Gesamtmenge an Bakterien). Vorzugsweise handelt es sich dabei um Bakterien des Ausgangsstamms, aus dem die Bakterienghosts hergestellt werden. Dabei sollte dieser Stamm nur gering pathogen oder attenuiert (abgeschwächt) sein. Außer dem Ausgangsstamm können ebenso lebende Bakterien der gleichen Art oder Gattung eingesetzt werden.

In einer weiteren bevorzugten Ausführungsform werden Bakterienghosts mit bis zu 50 %, vorzugsweise bis zu 10 % Bakterien, die als Lebendvakzine zugelassen sind (z. B. Salmonella- und Shigella-Stämme), gemischt und als Vakzin eingesetzt.

Die Impfung mit dem erfindungsgemäßen Vakzin oder den Vakzinkombinationen kann durch die dem Fachmann geläufigen Methoden erfolgen, beispielsweise intradermal, intramuskulär, intraperitoneal, intravenös, subkutan und intranasal.

Zur intramuskulären oder subkutanen Gabe kann das Vakzin beispielsweise in physiologischer Kochsalzlösung suspendiert sein. Zur intranasalen oder intraoccularen Applikation kann das Vakzin, beispielsweise in Form eines Sprays oder einer wäßrigen Lösung angewendet werden. Für lokale, beispielsweise orale Gabe ist es häufig erforderlich, die Immunogene zeitweise gegen Inaktivierung zu schützen, beispielsweise gegen saccharolytische Enzyme in der Mundhöhle oder gegen proteolytische Enzyme im Magen. Ein derartiger vorübergehender Schutz kann beispielsweise durch Verkapselung der Immunogene erfolgen. Diese Verkapselung kann beispielsweise durch Überziehen mit einem Schutzmittel (Mikroverkapselung) oder bei Einbettung einer Vielzahl von erfindungsgemäßen Immunogenen in einen schützenden Träger (Makroverkapselung) erfolgen.

Das Verkapselungsmaterial kann semipermiabel sein oder beim Einbringen in den menschlichen oder tierischen Körper semipermeabel werden. Üblicherweise wird für die Verkapselung eine biologisch abbaubare Substanz als Träger verwendet.

Die nachfolgenden Beispiele und die Abbildung erläutern die Erfindung weiter.

Fig.1 zeigt eine schematische Darstellung eines Bakterienghosts:
a) Längsschnitt durch ein gramnegatives Bakterium (om: äußere Membran; pp: periplasmatischer Raum, im: innere (cytoplasmatische) Membran, cp: Cytoplasma).
b) Ausbildung eines transmembranen Lysetunnels.
c) Durch den Lysetunnel ausströmendes Cytoplasma.

### Beispiel 1

### Fermentation und Lyse

Das Plasmid pMLl (Herstellung nach DE 40 05 874.3) wird in E. coli K12 (DSM 2093) integriert und die Kultur im Schüttelkolben bis zu OD 0,8 - 1,2 bei 600 nm angezogen, wobei die Expression des Lysegens E durch cI857 Repressormoleküle reprimiert ist (Eur. J. Biochem. 180 (1989) 393 bis 398). Durch Temperaturerhöhung auf 42°C während der exponentiellen Wachstumsphase der Bakterien erfolgt die Expression von Gen E durch thermische Inaktivierung der cI857 Repressormoleküle. Die durch Protein E verursachte Lyse von E. coli setzt je nach Kulturmedium (1 Voll- oder Minimalmedium, unter Belüftung im Schüttelwasserbad) der Bakterien zwischen 10 bis 30 min nach Temperaturerhöhung ein. Nach weiteren 10 bis 30 min ist die Lyse vollständig.

### Beispiel 2

### Modifizierte Protein E-Lyse.

Es wird wie in Beispiel 1 kultiviert, wobei jedoch 30 min. vor Temperaturerhöhung von 28°C auf 42°C das Kulturmedium durch Zugabe von Magnesiumsulfatlösung auf 0,2 mol/l Magnesiumsulfat gebracht wird. Dies verhindert trotz Expression von Gen E die Lyse der Bakterien.

30 min. nach Temperaturerhöhung werden die Zellen durch Zentrifugation geerntet. Durch Resuspension des Zellpellets in niedermolarem Puffer (PBS, 1 mmol/l Phosphatpuffer, 1 bis 10 mmol/l Tris - HCl pH 6 - 8) oder Wasser erfolgt eine sofortige Lyse der Zellen. Die dabei anfallenden Zellhüllen werden als Bakterienghosts bezeichnet. Bei diesen Bedingungen, die einer Kombination von Protein E-Lyse und osmotischem Schock entsprechen, wird eine größere Lysestruktur in den Bakterien erreicht. Die Morphologie der Bakterienghosts bleibt auch unter diesen Bedingungen weitgehend erhalten.

Zur Reinigung werden die Bakterienghosts 2 x mit PBS oder 0,9 % NaCl gewaschen (resuspendieren und zentrifugieren) und gefriergetrocknet.

### Beispiel 3

### Immunisierung

Je Maus werden 10⁹ gefriergetrocknete, in 0,9 % NaCl resuspendierte Keime (ensprechend 1 mg Bakterienghosts Trockengewicht) intraperitoneal 4 x in monatlichen Abständen zur Immunisierung gegeben. 8 Tage nach der letzten Immunisierung wird Serum gewonnen und die Antikörper werden isoliert. Es werden Antikörpertiter größer 1 : 1000 gegen Lipopolysaccharid und das äußere Membranprotein OmpF (J. Biol. Chem. 265 (1990) 6800 - 6810) erhalten.

Im T-Zell Proliferationstest nach Proc. Natl. Acad. Sci. USA 85 (1988) 6498 - 6502 ergibt sich ein T-Zell-Stimulationsindex von größer 20.

## Patentansprüche

1. Verfahren zur Herstellung eines Vakzins,
**dadurch gekennzeichnet,**
daß man ein gramnegatives Bakterium welches kein Fusionsproteingen enthält, das für eine hydrophobe, nicht lytisch wirkende membranintegrierende Proteindomäne und ein rekombinantes Protein codiert, mit einem Gen eines lytisch wirkenden Membranproteins aus Bakteriophagen, mit einem lytisch wirkenden Toxin-Releasegen oder mit Genen, die Teilsequenzen davon enthalten, die für lytische Proteine codieren, transformiert, das Bakterium kultiviert, das Lyse-Gen exprimiert und anschließend das so modifizierte Bakterium, dessen cytoplasmatische Bestandteile freigesetzt worden sind, wobei die Integrität des Zellwandkomplexes weitgehend erhalten bleibt, aus der Kulturbrühe isoliert.

2. Verfahren zur Herstellung eines Vakzins nach Anspruch 1,
**dadurch gekennzeichnet,**
daß bei der Kultivierung die Aktivität des lytisch wirkenden Membranproteins, des Toxin-Releasegens oder des Gens, das die genannten Teilsequenzen enthält, reprimiert wird und zu einem gewünschten Zeitpunkt der Kultivierung die Inhibierung oder Repression aufgehoben wird.

3. Verfahren zur Herstellung eines Vakzins nach Anspruch 1 oder 2,
**dadurch gekennzeichnet,**
daß als Lyse-Gen die DNA-Sequenz des E-Proteins, die N-terminale, membrandurchdringende Domäne des E-Proteins die DNA-Sequenz des L-Proteins, die C-terminale, membrandurchdringende Domäne des L-Proteins, die DNA-Sequenz des EL-Hybridproteins oder lytische Teilsequenzen davon verwendet werden.

4. Verfahren zur Herstellung eines Vakzins nach den Ansprüchen 1 bis 3,
**dadurch gekennzeichnet,**
daß das modifizierte Bakterium mit pharmazeutischen Hilfs-oder Trägerstoffen gemischt wird.

5. Verfahren zur Herstellung eines Vakzins nach den Ansprüchen 1 bis 4,
**dadurch gekennzeichnet,**
daß das modifizierte Bakterium in einer antigenhaltigen Lösung suspendiert wird.

6. Verfahren zur Herstellung eines Vakzins nach den Ansprüchen 1 bis 5,
**dadurch gekennzeichnet,**
daß das modifizierte Bakterium mit einem Anteil von 0,01 % bis 5 % an lebenden, gering pathogenen oder attenuierten Bakterien gemischt wird.

7. Verfahren nach den Ansprüchen 1 bis 6,
**dadurch gekennzeichnet,**
daß das modifizierte Bakterium mit einem Anteil von 0,01 % bis 2 % an lebenden, gering pathogenen oder attenuierten Bakterien gemischt wird.

8. Verfahren zur Herstellung eines Vakzins nach den Ansprüchen 1 bis 6,
**dadurch gekennzeichnet,**
daß das modifizierte Bakterium mit einem Anteil von 0,01 % bis 1 % an lebenden, gering pathogenen oder attenuierten Bakterien gemischt wird.

## Claims

1. Process for the production of a vaccine,
**wherein**
a gram-negative bacterium which contains no fusion protein gene that codes for a hydrophobic, nonlytically active membrane-integrating protein domain and for a recombinant protein is transformed with a gene of a lytically-active membrane protein from bacteriophages or with a lytically active toxin release gene or with genes which contain partial sequences thereof coding for lytic proteins, culturing the bacterium, expressing the lysis gene and subsequently isolating the bacterium modified in this way from the culture broth whose cytoplasmic components have been released while the integrity of the cell wall complex is substantially retained.

2. Process for the production of a vaccine as claimed in claim 1,
**wherein**
the activity of the lytically active membrane protein, the toxin release gene or the gene which contains the said partial sequences is repressed during culture and the inhibition or repression is abolished at a desired time during culture.

3. Process for the production of a vaccine as claimed in claim 1 or 2,
**wherein**
the DNA sequence of the E-protein, the N-terminal, membrane-spanning domain of the E-protein, the DNA sequence of the L-protein, the C-terminal, membrane-spanning domain of the L-protein or the DNA sequence of the EL-hybrid protein or lytically-active partial sequences thereof are used as the lysis gene.

4. Process for the production of a vaccine as claimed in claims 1 to 3,
**wherein**
the modified bacterium is mixed with pharmaceutical auxiliary substances or carrier materials.

5. Process for the production of a vaccine as claimed in claims 1 to 4,
**wherein**
the modified bacterium is suspended in an antigen-containing solution.

6. Process for the production of a vaccine as claimed in claims 1 to 5,
**wherein**
the modified bacterium is mixed with a portion of 0.01 % - 5 % live, slightly pathogenic or attenuated bacteria.

7. Process as claimed in claims 1 to 6,
**wherein**
the modified bacterium is mixed with a portion of 0.01 % - 2 % live, slightly pathogenic or attenuated bacteria.

8. Process for the production of a vaccine as claimed in claims 1 to 6,
**wherein**
the modified bacterium is mixed with a portion of 0.01 % - 1 % live, slightly pathogenic or attenuated bacteria.

## Revendications

1. Procédé pour la préparation d'un vaccin, caractérisé en ce que l'on transforme une bactérie Gram négatif, ne contenant aucun gène de protéine en fusion, qui code pour un domaine protéïnique intégrateur de membrane à action non lytique et code une protéine recombinante, avec un gène d'une protéine de membrane à action lytique à partir d'un gène bactériophage, avec un gène de libération de toxine à action lytique ou avec des gènes qui contiennent des séquences partielles qui codent pour les protéines lytiques, en ce que l'on cultive la bactérie qui exprime le gène lyse et consécutivement en ce que l'on isole à partir du bouillon de culture la bactérie ainsi modifiée dont les composants cytoplasmatiques ont été libérés, l'intégrité du complexe des parois cellulaires demeurant conservée dans une large mesure.

2. Procédé pour la préparation d'un vaccin selon la revendication 1, caractérisé en ce que lors de la mise en culture, on réprime l'activité de la protéine de membrane à action lytique, du gène de libération de toxine ou du gène qui contient les séquences partielles citées, et en ce qu'à un moment souhaité de la culture on supprime l'inhibition ou la répression.

3. Procédé pour la préparation d'un vaccin selon la revendication 1 ou 2, caractérisé en ce qu'en tant que gène lyse, on utilise la séquence A.D.N. de la protéine E, les terminaisons N, le domaine de pénétration de membrane de la protéine E, la séquence A.D.N. de la protéine N, les terminaisons C, les domaines de pénétration de membrane de la protéine N, la séquence A.D.N. de la protéine hybride LE ou les séquences partielles lytiques.

4. Procédé pour la préparation d'un vaccin selon les revendications 1 à 3, caractérisé en ce que la bactérie modifiée est mélangée avec des substances pharmaceutiques auxiliaires ou porteuses.

5. Procédé pour la préparation d'un vaccin selon les revendications 1 à 4, caractérisé en ce que la bactérie modifiée est mise en suspension dans une solution contenant un antigel.

6. Procédé pour la préparation d'un vaccin selon les revendications 4 et 5, caractérisé en ce que la bactérie modifiée est mélangée avec une portion de 0,01 % jusqu'à 5 % de bactéries vivantes, légèrement pathogènes ou atténuées.

7. Procédé selon les revendications 1 à 6, caractérisé en ce que la bactérie modifiée est mélangée avec une portion de 0,01 % jusqu'à 2 % de bactéries vivantes, légèrement pathogènes ou atténuées.

8. Procédé pour la préparation d'un vaccin selon les revendications 1 à 6, caractérisé en ce que la bactérie modifiée est mélangée avec une portion de 0,01 % à 1 % de bactéries vivantes, légèrement pathogènes ou atténuées.
